# EUROPEAN PATENT SPECIFICATION

(11) **EP 3 050 591 B1**
(45) Date of publication and mention of the grant of the patent: **19.09.2018**
(21) Application number: 14849980.9
(22) Date of filing: 22.09.2014
(51) Int. Cl.: A61M 39/10, A61M 5/162, A61M 5/24, A61M 5/28, A61J 1/20, A61M 5/31

(54) **SYRINGE OUTER CYLINDER WITH PUNCTURE FUNCTION, SYRINGE WITH PUNCTURE FUNCTION, AND PREFILLED SYRINGE WITH PUNCTURE FUNCTION**
AUSSENZYLINDER EINER SPRITZE MIT DURCHSTECHFUNKTION, SPRITZE MIT DURCHSTECHFUNKTION UND VORGEFÜLLTE SPRITZE MIT DURCHSTECHFUNKTION
CYLINDRE EXTERNE DE SERINGUE PRÉSENTANT UNE FONCTION DE PERFORATION, SERINGUE PRÉSENTANT UNE FONCTION DE PERFORATION ET SERINGUE PRÉREMPLIE PRÉSENTANT UNE FONCTION DE PERFORATION

(30) Priority: 27.09.2013 JP 2013202148
(43) Date of publication of application: 03.08.2016
(73) Proprietor: Terumo Kabushiki Kaisha, Tokyo 151-0072 (JP)
(72) Inventor: HORIUCHI, Aiko, Ashigarakami-gun Kanagawa 259-0151 (JP); FUKE, Shigeaki, Fujinomiya-shi Shizuoka 418-0004 (JP); MORIYAMA, Yoshihisa, Ashigarakami-gun Kanagawa 259-0151 (JP)
(74) Representative: Casalonga
(86) International application number: PCT/JP2014/075133
(87) International publication number: WO 2015/046170

(56) References cited:
- JP-A- H10 152 566
- JP-A- 2003 024 439
- JP-A- 2004 160 206
- JP-A- 2004 160 206
- JP-A- 2011 521 693
- JP-B2- 4 871 492
- US-A- 5 071 413

## Description

### Technical Field

The present invention is defined in the appended claims, and relates to a syringe barrel with puncture function having a puncture function for connection with a medical device, a syringe with puncture function, and a prefilled syringe with puncture function.

### Background Art

In recent years, prefilled syringes have been widely used. A prefilled syringe is filled with a medical liquid such as a liquid medication or a solvent for medication. The prefilled syringe filled with a solvent for medication is used for administration of powdered formulation that needs to be dissolved before use, such as lyophilized formulation. The prefilled syringe filled with a solvent for medication is connected to a medication container (e.g., vial) storing the powdered formulation, the solvent in the syringe is injected into the medication container, the powdered formulation in the container is dissolved in the solvent, and the solution is withdrawn into the syringe for administration. A metal needle is generally used to connect the prefilled syringe filled with a solvent for medication, to the medication container storing the powdered formulation, but a product using a dedicated connector has been recently proposed. However, even if connector is used, it is troublesome to remove and change the metal needle or connector mounted to the syringe with an injection needle or the like for dissolution, before administration.

In order to remove the troublesomeness as described above, the following is disclosed in JP 3103938 (Patent Literature 1) .

A syringe has a distal end including a tubular port section made of a single, integrally molded plastic piece, which is capable of both connection with female luer connectors and penetration of resealable-diaphragm connection sites. The tubular port section includes a tubular member defining a first tubular section having a distal end capable of resealably penetrating a latex resealable-diaphragm injection site, and aperture means adjacent the distal end. The tubular member also defines a frustoconical male luer section positioned proximally of the first tubular section and proportioned to be sealable with female luer connectors, and the male luer section has a minimum diameter that is greater than the maximum diameter of the first tubular section. The male luer section defines a 6/100 taper conforming to ISO standards, and has an outer diameter at end of 3.925 mm to 4.027 mm.

### Citation List

### Patent Literature

Patent Literature 1: Japanese Patent No. 3103938
Patent Literature 2: US 5 071 413 A

### Summary of Invention

### Technical Problem

The syringe disclosed in Patent Literature 1 can be used as the prefilled syringe filled with a solvent for dissolving powdered formulation or the like, and the first tubular section can pierce the medication container (e.g., vial) storing a powdered formulation to connect the prefilled syringe to the medication container. The solvent in the syringe is injected into the medication container, the powdered formulation in the container is dissolved in the solvent to be withdrawn into the syringe, the injection needle or the like is connected to the male luer section, and liquid medication in which the powdered formulation is dissolved is capable of being administered. However, the syringe of Patent Literature 1 has the male luer section conforming to the ISO standards and positioned proximally of the first tubular section. Thus, when the male luer section connected to a connection portion (e.g., hub) having a female type luer taper conforming to the ISO standards, such as an injection needle, or a three-way stopcock, the first tubular section interferes with the inside of the connection portion, before the male luer section is liquid-tightly connected to the connection portion, and the liquid-tight connection may be prevented.

An object of certain embodiments of the present invention is to provide a syringe barrel with puncture function which is liquid-tightly connected to a connection portion (hub) having a female type luer taper (hereinafter, simply referred to as "female luer taper") conforming to the ISO standard 594-1 in a syringe prepared for administration, and can pierce a puncturable portion of a medication container (vial), a syringe with puncture function using the syringe barrel, and a prefilled syringe with puncture function using the syringe.

### Solution to Problem

The above object is achieved by the following.

A syringe barrel with puncture function including a cylindrical main body portion configured to allow liquid-tight sliding of a gasket, and a hollow distal end portion configured to extend distally from the cylindrical main body portion. The distal end portion of the syringe barrel includes a hollow small-diameter distal end portion having a puncture end portion configured to be inserted into a puncturable portion of a medical device, and a large-diameter portion having a diameter larger than that of the small-diameter distal end portion. The outer diameter of the large-diameter portion is increased from a distal end to a proximal end thereof by a 6/100 taper. The distal end of the large-diameter portion has an outer diameter of 4.04 to 4.20 mm, and the small-diameter distal end portion has a length of 5 to 6 mm. The large-diameter portion is to be used to connect portion of the medical device, which has a female luer taper. Upon connection of the large-diameter portion to the connection portion, the small-diameter distal end portion is housed in the connection portion, and the larger-diameter portion has a length of 5 to 9 mm.

Further, the above object is achieved by the following.

A syringe with puncture function including the syringe barrel with puncture function, a gasket configured to be slidably housed in the syringe barrel, and a pusher configured to be mounted to or mountable to a rear end of the gasket.

Further, the above object is achieved by the following.

A prefilled syringe with puncture function including the syringe with puncture function, a medical liquid filled in the syringe barrel, and a sealing cap configured to seal the distal end portion of the syringe barrel.

### Brief Description of Drawings

Fig. 1 is a front view of a prefilled syringe with puncture function using a syringe barrel with puncture function according to an embodiment of the present invention.
Fig. 2 is an enlarged cross-sectional view of a distal end portion of the prefilled syringe with puncture function illustrated in Fig. 1.
Fig. 3 is a front view of the prefilled syringe with puncture function illustrated in Fig. 1, from which a sealing cap is removed.
Fig. 4 is a cross-sectional view taken along the line A-A of Fig. 3.
Fig. 5 is a front view of the prefilled syringe illustrated in Fig. 3 in which a lock adapter is proximally moved.
Fig. 6 is a cross-sectional view taken along the line B-B of Fig. 5.
Fig. 7 is an enlarged front view of a distal end side portion of the syringe barrel with puncture function according to an embodiment of the present invention from which the lock adapter is removed.
Fig. 8 is a perspective view of the syringe barrel with puncture function according to an embodiment of the present invention.
Fig. 9 is a perspective view of the syringe barrel with puncture function according to the present invention in which the lock adapter is proximally moved.
Fig. 10 is a front view of the lock adapter used for the syringe barrel with puncture function according to an embodiment of the present invention.
Fig. 11 is a bottom view of the lock adapter illustrated in Fig. 10.
Fig. 12 is a cross-sectional view taken along the line C-C of Fig. 10.
Fig. 13 is an enlarged cross-sectional view of a distal end portion of the syringe barrel with puncture function according to an embodiment of the present invention.
Fig. 14 is an enlarged cross-sectional view of the distal end portion of the syringe barrel with puncture function according to an embodiment of the present invention in which the lock adapter is proximally moved.
Fig. 15 is a schematic diagram illustrating the syringe barrel with puncture function according to an embodiment of the present invention in which a connection portion of a medical device having a female luer taper is mounted to the distal end portion.
Fig. 16 is a schematic diagram illustrating operation of the syringe barrel with puncture function and the prefilled syringe with puncture function according to an embodiment of the present invention.
Fig. 17 is a schematic diagram illustrating operation of the syringe barrel with puncture function and the prefilled syringe with puncture function according to an embodiment of the present invention.
Fig. 18 is a schematic diagram illustrating operation of the syringe barrel with puncture function and the prefilled syringe with puncture function according to an embodiment of the present invention.
Fig. 19 is a schematic diagram illustrating operation of the syringe barrel with puncture function and the prefilled syringe with puncture function according to an embodiment of the present invention.

### Description of Embodiments

A syringe barrel with puncture function, a syringe with puncture function, and a prefilled syringe with puncture function according to an embodiment of the present invention will be described using an example illustrated in the drawings.

A syringe barrel 3 with puncture function includes a cylindrical main body portion 30 in which a gasket 4 is liquid-tightly slidable, and a hollow distal end portion 31 extending distally from the cylindrical main body portion 30.

The distal end portion 31 of the syringe barrel 3 includes a hollow small-diameter distal end portion 32 having have a puncture end portion 37 configured to be inserted into a puncturable portion of another medical device, and a large-diameter portion 33 having a diameter larger than that of the small-diameter distal end portion 32. The outer diameter of the large-diameter portion 33 is increased from a distal end to a proximal end thereof by a 6/100 taper. The distal end of the large-diameter portion 33 has an outer diameter of 4.04 to 4.20 mm, and the small-diameter distal end portion 32 has a length of 5 to 6 mm. The small-diameter distal end portion 32 can be used to puncture a puncturable portion of the other medical device, and the large-diameter portion 33 can be used to make a liquid-tight connection with a connection portion of the other medical device having a female luer taper. The small-diameter distal end portion 32 is housed in the connection portion, upon connection of the large-diameter portion 33 to the connection portion.

A syringe 2 with puncture function includes the syringe barrel 3 with puncture function, the gasket 4 slidably housed in the syringe barrel 3, and a pusher 5 mounted to or mountable to a rear end of the gasket 4.

A prefilled syringe 1 with puncture function includes the syringe 2 with puncture function, a medical liquid 7 contained in the syringe barrel 3, and a sealing cap 8 sealing the distal end portion 31 of the syringe barrel 3.

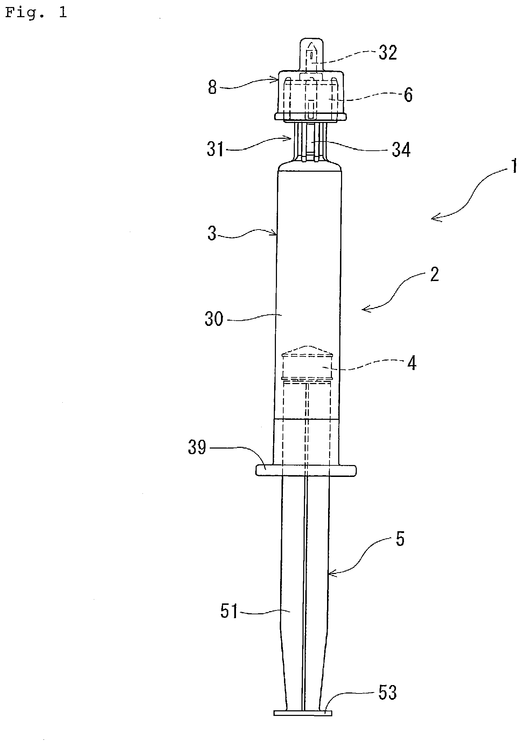

As illustrated in Figs. 1 to 6, the prefilled syringe 1 with puncture function according to the present example includes the syringe 2 with puncture function, the medical liquid 7 filled in the syringe barrel 3, and the sealing cap 8 configured to seal the distal end portion 31 of the syringe barrel 3.

The syringe 2 with puncture function includes the syringe barrel 3 with puncture function, the gasket 4 slidably housed in the syringe barrel 3, and the pusher 5 mounted to or mountable to the rear end of the gasket 4.

As illustrated in Figs. 1 to 3, the syringe barrel 3 with puncture function has the cylindrical main body portion 30 in which the gasket 4 is liquid-tightly slidable, the hollow distal end portion 31 extending distally from the cylindrical main body portion 30, and a flange portion 39 provided at a proximal end portion of the cylindrical main body portion 30. The syringe barrel 3 with puncture function according to the present invention has puncture function, not for directly inserting into a human body, but for connection with a medical device.

As illustrated in Figs. 1 to 9, the syringe barrel 3 has the cylindrical main body portion 30, the distal end portion 31 extending from a distal end of the cylindrical main body portion 30, and a distal end provided with the puncture end portion 37.

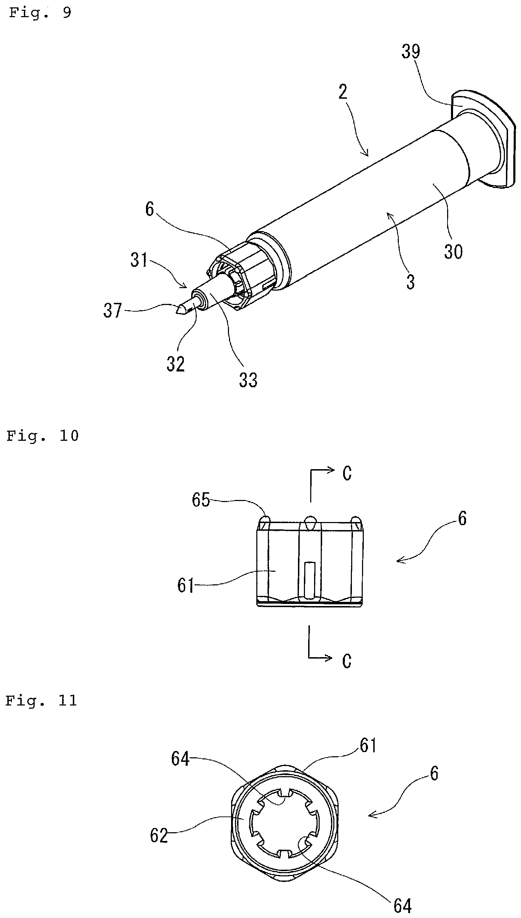

Especially, as illustrated in Fig. 9, the distal end portion 31 has a hollow small-diameter distal end portion 32 having the puncture end portion 37 configured to pierce the puncturable portion (e.g., elastic sealing portion of vial) of another medical device (e.g., vial), and a large-diameter portion 33 extending to a proximal end of the small-diameter distal end portion 32, and having a diameter larger than that of the small-diameter distal end portion 32. A boundary (distal end surface of the large-diameter portion) between the proximal end of the small-diameter distal end portion 32 and the large-diameter portion 33 is defined as a stepped portion. The distal end surface of the large-diameter portion 33 being the stepped portion is defined as an annular flat surface to inhibit insertion of the distal end portion 31 into the puncturable portion of the medical device, as described later. Furthermore, the large-diameter portion 33 is formed to have the outer diameter increased from the distal end to the proximal end thereof by a 6/100 taper, and to be connected to the connection portion (hub, port) of another medical device (e.g., injection needle, three-way stopcock, infusion line, etc.) having the female luer taper.

The outer diameter X of the distal end of the large-diameter portion 33 is 4.0 to 4.2 mm. Since the outer diameter of the distal end of the large-diameter portion 33 is larger than that of the connection portion having a male luer taper conforming to the ISO standard 594-1, the large-diameter portion 33 can be mounted to the connection portion of the medical device having the female luer taper, but a length of the large-diameter portion 33 inserted into the medical device is small. Therefore, when the large-diameter portion 33 is liquid-tightly connected to the connection portion having the female luer taper, some space remains in the connection portion distally from the large-diameter portion 33. The outer diameter X of the distal end of the large-diameter portion 33 is more preferably 4.04 to 4.16 mm. Thus, the large-diameter portion 33 is inserted into the connection portion having the female luer taper by a sufficient length, and liquid-tight connection can be accurately obtained. Furthermore, the large-diameter portion 33 preferably has a length of 5 to 9 mm. Thus, the liquid-tight connection can be obtained further accurately. Furthermore, the small-diameter distal end portion 32 has a length Y of 5 to 6 mm. Thus, the small-diameter distal end portion 32 can pierce an elastic sealing portion or the like of a normal vial, and upon connection to the connection portion having the female luer taper, the small-diameter distal end portion 32 can be housed in the space formed in the connection portion distally from the distal end of the large-diameter portion.

The small-diameter distal end portion 32 includes the puncture end portion 37 formed by a closed pointed end. The puncture end portion 37 has a reduced diameter to provide a conical shape, and has a distal end defined as a puncture end. Two opposed lateral side holes 36 communicating between the inside and outside of the small-diameter distal end portion 32 are provided near the puncture end portion 37. Furthermore, the distal end portion 31 has an inside space communicating between the outside of the distal end portion 31 through the lateral side holes 36.

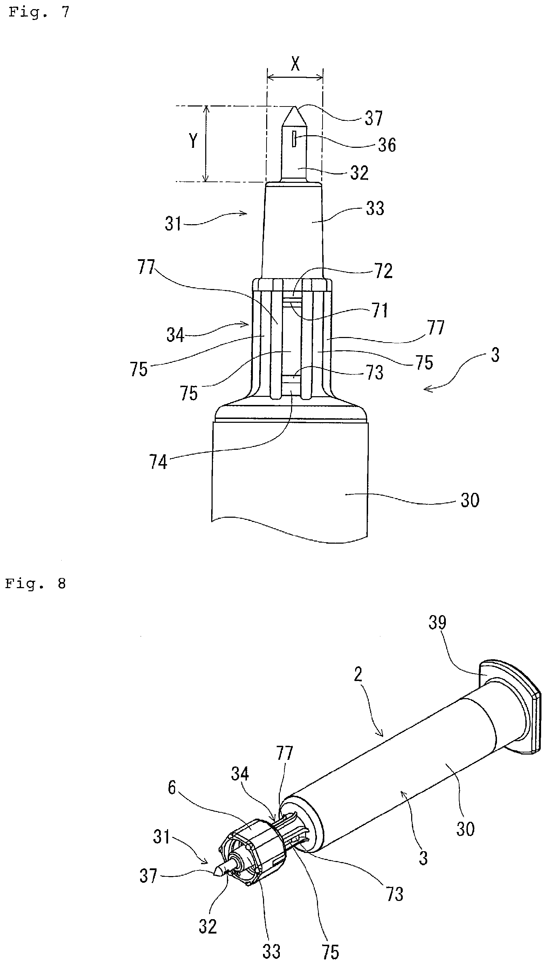

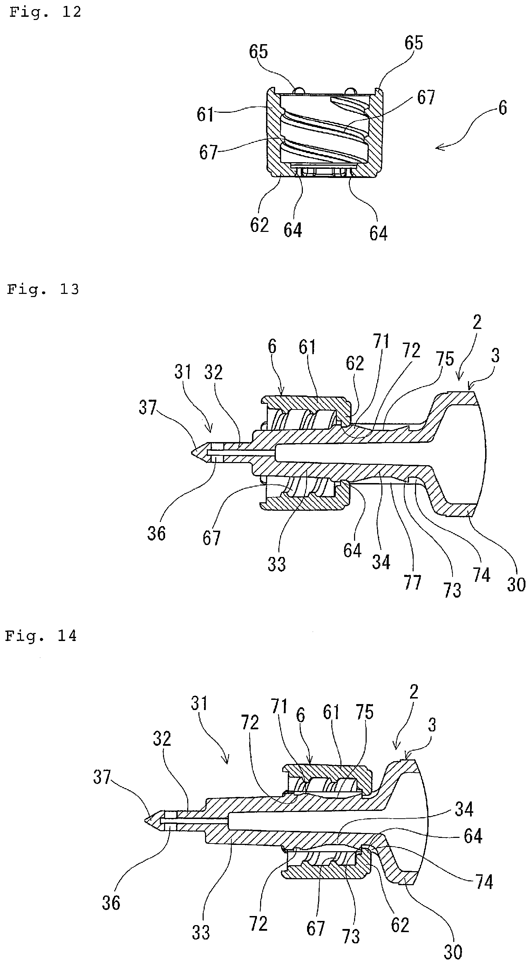

As illustrated in Fig. 7, in the cylindrical main body portion 30 according to the present example, the distal end portion 31 includes a lock adapter sliding portion 34 provided between the cylindrical main body portion 30 and the large-diameter portion 33. The lock adapter 6 described later is slidably mounted to the sliding portion 34. The sliding portion 34 is formed to have a diameter slightly larger than that of the proximal end of the large-diameter portion 33. As illustrated in Figs. 7 and 13, the sliding portion 34 includes a large number of sliding grooves 75 configured to axially extend and a large number of lock adapter rotation restriction ribs 77 similarly configured to axially extend. Each of the sliding grooves 75 is formed between the rotation restriction ribs 77. The sliding groove 75 has a starting end at a part slightly proximal relative to a distal end of the sliding portion 34, and terminates at a shoulder portion (distal end portion of the barrel body portion 30) of the syringe barrel 3. Furthermore, in the syringe barrel 3 according to the present example, a rib 71 and a rib 73 are provided in one or more sliding grooves 75. The rib 71 comprises a first holding mechanism for holding the lock adapter 6 so that the lock adapter 6 substantially covers the large-diameter portion 33, and the rib 73 comprises a second holding mechanism for holding the lock adapter 6 so that the large-diameter portion 33 is substantially exposed. The rib 71 comprising the first holding mechanism is provided at a position slightly proximal relative to the distal end of the sliding groove 75, and a recessed portion 72 is formed between the rib 71 and the distal end portion of the sliding portion 34. The rib 73 comprising the second holding mechanism is provided at a position slightly distal relative to the proximal end of the sliding groove 75, and a recessed portion 74 is formed between the rib 73 and a distal end surface of the shoulder portion of the cylindrical main body portion 30 of the barrel.

A material comprising the syringe barrel 3 includes a resin, for example, polypropylene, polyethylene, polystyrene, polyamide, polycarbonate, polyvinyl chloride, poly(4-methyl pentene-1), an acrylic resin, an acrylonitrile-butadiene-styrene copolymer, polyester such as polyethylene terephthalate, a cyclic olefin polymer, and a cyclic olefin copolymer. However, in particular, the resin such as polypropylene, the cyclic polyolefin polymer, or the cyclic olefin copolymer is preferably employed for easy moldability and heat resistance. As the material comprising the barrel 3, the cyclic polyolefin polymer or the cyclic olefin copolymer is particularly preferably employed. The cyclic olefin polymer or the cyclic olefin copolymer has high transparency to visually confirm, from outside the liquid medication contained therein, , and has heat resistance to withstand autoclave sterilization.

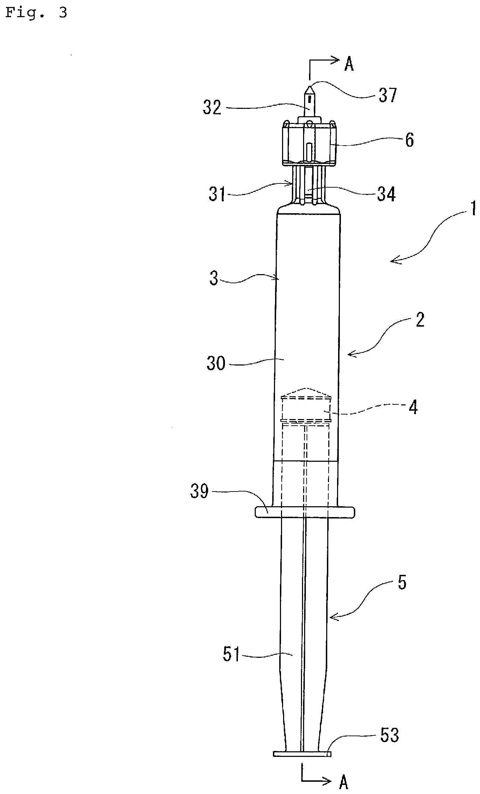

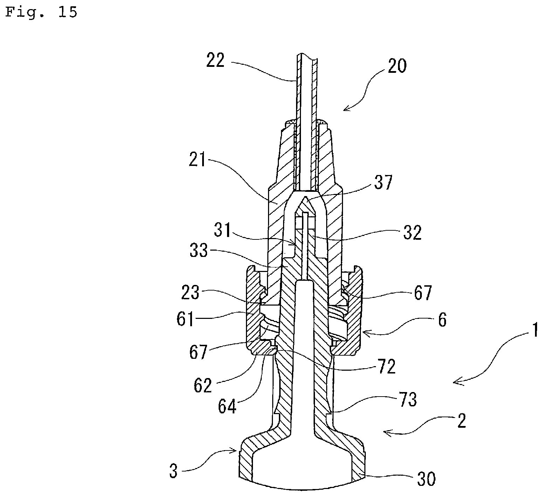

As illustrated in Figs. 3 to 6 and Figs. 8 to 12, the syringe barrel 3 according to the present example includes the lock adapter 6 configured to hold the connection portion (hub 21) of the medical device (e.g., injection needle 20 of Fig. 15) connected to the distal end portion 31 of the syringe barrel 3.

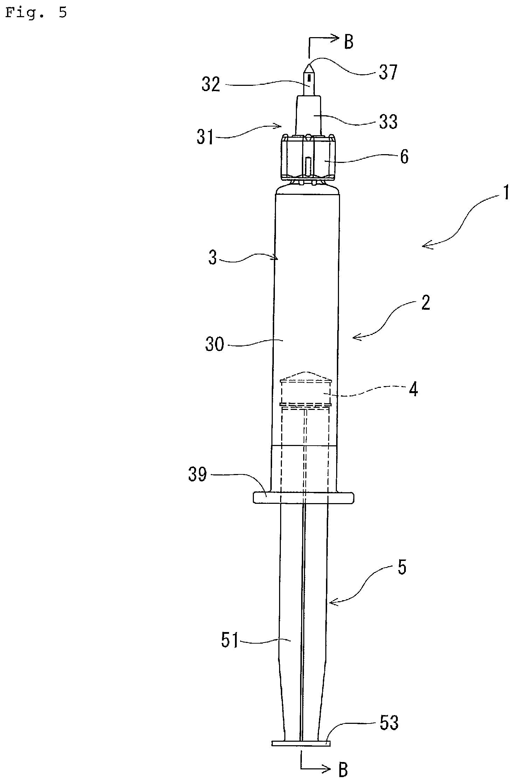

The lock adapter 6 is axially slidable from a state in which the lock adapter 6 substantially covers the large-diameter portion 33 (state illustrated in Figs. 3, 4, and 8) to a state in which the large-diameter portion 33 is substantially exposed (state illustrated in Figs. 5, 6, and 9). Further, in this example, the lock adapter 6 is mounted to the sliding portion 34 in a slidable and non-rotatable manner. Still further, the lock adapter 6 and the sliding portion 34 includes the first holding mechanism configured to hold the lock adapter 6 in a state in which the lock adapter 6 substantially covers the large-diameter portion 33, and the second holding mechanism configured to hold the lock adapter 6 in a state in which the large-diameter portion 33 is substantially exposed. As illustrated in Figs. 12 to 15, the lock adapter 6 includes, on an inner surface thereof, an adapter-side engagement portion 67 engageable with a connection-side projection portion (e.g., rear end external rib 23 of the hub 21 of the injection needle 20 of Fig. 15) provided at the connection portion of the medical device.

Specifically, as illustrated in Figs. 10 to 14, the lock adapter 6 has a cylindrical main body portion 61 an annular projection portion 62 provided on an inner surface of a proximal end portion of the cylindrical main body portion 61, and a plurality of projections 64 configured to project inward from the annular projection portion 62. Each of the projections 64 of the lock adapter 6 is stored in the sliding groove 75 of the sliding portion 34 of the syringe barrel 3. Further, the projections 64 abut on the lock adapter rotation restriction ribs 77 of the sliding portion 34, and rotation of the lock adapter is restricted. In the state of Fig. 13 in which the lock adapter 6 substantially covers the large-diameter portion 33, the projection 64 of the lock adapter 6 is positioned at the recessed portion 72 formed between the rib 71 comprising the first holding mechanism of the syringe barrel 3 and the distal end portion of the sliding portion 34, and the projection 64 is engaged with the rib 71, holding the engagement. Thus, in the syringe barrel 3 according to the present example, the first holding mechanism holds the lock adapter 6 so that the lock adapter 6 substantially covers the large-diameter portion 33, and the first holding mechanism includes the projection 64 of the lock adapter 6, and the rib 71 of the sliding portion 34. Further, in the state of Fig. 14 in which the large-diameter portion 33 of the syringe barrel 3 is substantially exposed, the projection 64 of the lock adapter 6 is positioned at the recessed portion 74 formed between the rib 73 comprising the second holding mechanism of the syringe barrel 3 and the distal end of the shoulder portion of the cylindrical main body portion 30 of the barrel, and the projection 64 is engaged with the rib 73, holding the engagement. Thus, in the syringe barrel 3 according to the present example, the second holding mechanism holds the lock adapter 6 so that the large-diameter portion 33 of the syringe barrel 3 is substantially exposed, and the second holding mechanism includes the projection 64 of the lock adapter 6, and the rib 73 of the sliding portion 34.

The lock adapter 6 has an inner peripheral surface on which the adapter-side thread engagement portion (cylindrical main body portion-side thread engagement portion) 67 is formed. In the present example, the thread engagement portion 67 includes two helical ribs. The adapter-side thread engagement portion 67 is threadedly engaged with a medical device (e.g., a rib or a thread engagement portion formed on a flange of an injection needle, a connector, a three-way stopcock or the like) connected to the large-diameter portion 33 of the syringe barrel. Further, the lock adapter 6 has a distal end surface on which a plurality of projections 65 are formed.

A material comprising the lock adapter 6 and the cap includes for example a resin, such as polypropylene, polyethylene, polystyrene, polyamide, polycarbonate, polyvinyl chloride, poly (4-methyl pentene-1), an acrylic resin, an acrylonitrile-butadiene-styrene copolymer, polyester such as polyethylene terephthalate, or cyclic polyolefin. In particular, the resin such as polypropylene or cyclic polyolefin is preferably employed for easy moldability and heat resistance.

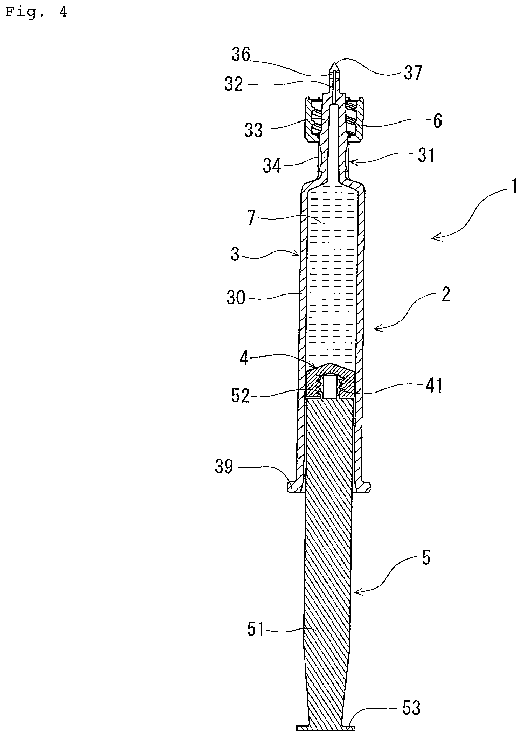

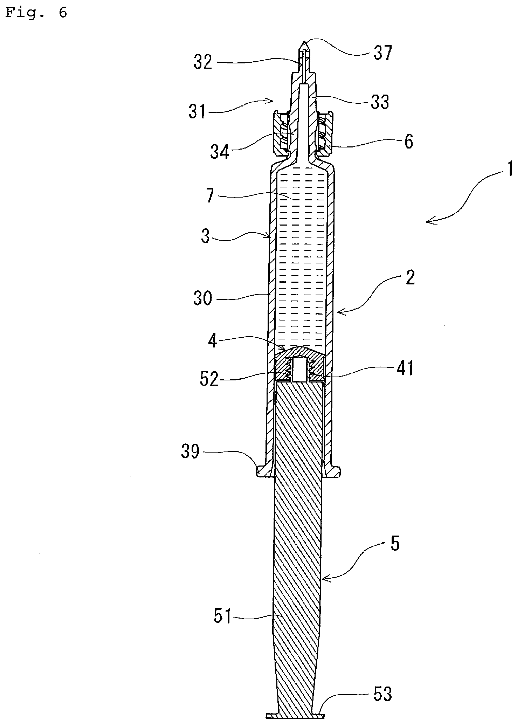

As illustrated in Figs. 4 and 6, the gasket 4 includes a main body portion extending to have substantially the same outer diameter, and a plurality of annular ribs provided in the main body portion, and the ribs make liquid-tight contact with an inner surface of the barrel 3. In the present example, there are two annular ribs. In other examples, an appropriate number of annular ribs not less than two may be employed as long as requirements for the liquid tightness and the slidability are satisfied. The gasket 4 has a distal end surface of a shape corresponding to the shape of an inner surface of the distal end of the main body portion 30 of the syringe barrel 3 so that a gap is not formed between the gasket and the barrel as much as possible, when the distal end surface of the gasket 4 abuts on the inner surface of the distal end of the barrel 3. A material comprising the gasket 4 preferably includes elastic rubber (e.g., butyl rubber, latex rubber, silicone rubber), a synthetic resin (e.g., a styrenic elastomer such as SBS elastomer or SEBS elastomer, or a polyolefinic elastomer such as ethylene-α-olefin copolymer elastomer), or the like.

The gasket 4 is provided with a recessed portion extending inwardly from the rear end portion of the gasket 4, and the recessed portion is provided with a plunger mounted portion 41. In the present example, the plunger mounted portion 41 is defined as a female threaded portion.

A plunger (pusher) 5 includes a main body portion 51 configured to have a cross-shaped cross-section and axially extending, a gasket mounted portion 52 configured to project from the main body portion 51, and a pressing disk portion 53 provided at a rear end portion. The gasket mounted portion 52 includes a projection portion, and a male threaded portion formed on an outer surface of the projection portion. The plunger 5 may be separately provided, and mounted when used.

The medical liquid 7 to be filled in the prefilled syringe 1 may include a solvent for medication, such as, distilled water for injection, normal saline, or glucose solution, and/or further a medication (e.g., vitamin preparation, minerals, cyclosporine, benzodiazepine medication, sodium chloride injection solution, antibiotics, protein preparation).,The liquid medication can dissolve a powdered formulation contained in a medication container.

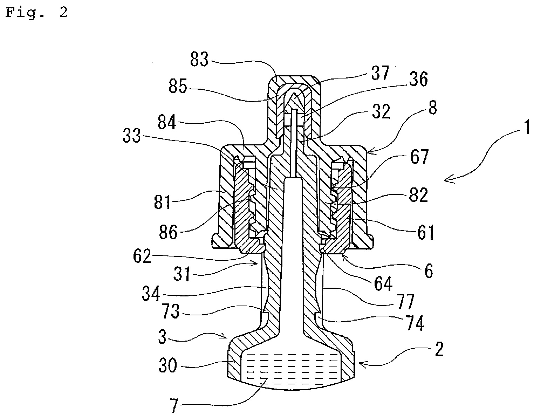

Next, the sealing cap 8 will be described using Fig. 2.

As illustrated in Figs. 1 and 2, the sealing cap 8 covers a distal end side portion (the small-diameter distal end portion, the large-diameter portion) of the distal end portion of the syringe barrel 3. In particular, the sealing cap 8 according to the present example is mounted to the distal end portion of the syringe barrel 3 configured so that the lock adapter 6 substantially covers the large-diameter portion 33 of the syringe barrel 3, and the sealing cap 8 seals the distal end portion of the syringe barrel 3. Thus, the sealing cap 8 covers the small-diameter distal end portion of the syringe barrel 3 and the lock adapter, as illustrated in Figs. 1 and 2.

Therefore, the sealing cap 8 includes a lock adapter housing portion 81, a closed small-diameter distal end portion housing portion 83, a large-diameter portion housing portion 82, and a disk-shaped connection portion 84 configured to connect the above portions. The small-diameter portion housing portion 83 extends distally from the center of the disk-shaped connection portion 84, and is formed into a short, small cylinder portion having a closed distal end. Further, a cylindrical sealing member 85 is housed in the small-diameter portion housing portion 83. The cylindrical sealing member 85 has an annular inside surface sealing the lateral side holes 36 in the small-diameter distal end portion 32. In the present example, the cylindrical sealing member 85 has a closed distal end. The cylindrical sealing member 85 is preferably an elastic member to liquid-tightly seal the lateral side holes 36 in the small-diameter distal end portion 32. A material comprising the cylindrical sealing member preferably includes for example natural rubber, a synthetic rubber such as isoprene rubber, butadiene rubber, fluoro-rubber, silicone rubber, or a thermoplastic elastomer such as an olefinic elastomer or styrenic elastomer.

The large-diameter portion housing portion 82 is provided at the center of the sealing cap 8, and has a cylindrical shape. The large-diameter portion housing portion 82 has an inner diameter formed slightly larger than the outer diameter of the large-diameter portion 33. The inner diameter is configured to be slightly increased toward the proximal end from the distal end into a tapered shape, and accommodates the whole nozzle portion from a proximal opening. The large-diameter portion housing portion 82 has an outer surface on which a cap-side thread engagement portion 86 is formed for thread engagement with the lock adapter-side thread engagement portion 67 formed on the inner surface of the lock adapter 6. Thus, the lock adapter 6 and the sealing cap 8 are threadedly engaged with each other between the outer surface of the large-diameter portion housing portion 82 and the inner surface of the lock adapter 6.

The lock adapter housing portion 81 is formed to surround the large-diameter portion housing portion 82, has a cylindrical body having a closed distal end, and houses the lock adapter 6 between an inner surface of the lock adapter housing portion and the outer surface of the large-diameter portion housing portion. Furthermore, the lock adapter housing portion 81 formed into a cylindrical shape is positioned concentrically with the large-diameter portion housing portion 82. The lock adapter housing portion 81 has a substantially constant inner diameter from the distal end to the proximal end. The sealing cap 8 preferably has an outside surface vertically knurled to prevent slippage of fingers upon rotation of the sealing cap.

A material comprising the sealing cap includes for example a resin, such as polypropylene, polyethylene, polystyrene, polyamide, polycarbonate, polyvinyl chloride, poly(4-methyl pentene-1), an acrylic resin, an acrylonitrile-butadiene-styrene copolymer, polyester such as polyethylene terephthalate, or a cyclic polyolefin. In particular, the resin such as a polypropylene or a cyclic polyolefin is preferably employed for easy moldability and heat resistance.

The sealing cap may include only the elastic member. In this configuration, the cylindrical sealing member and the cap-side thread engagement portion 86 of the large-diameter portion housing portion 82 are omitted, the small-diameter distal end portion housing portion 83 seals the lateral side holes 36 in the small-diameter distal end portion 32, and an inner surface of the large-diameter portion housing portion 82 fits to the outer surface of the large-diameter portion 33. In the sealing cap of this type, the lock adapter housing portion 81 can be also omitted. The sealing cap of this type preferably has a grip portion in which a distal end portion of the sealing cap has a diameter larger than those of other portions. Such a configuration facilitates removal of the sealing cap. Furthermore, while the sealing cap is mounted, the lock adapter 6 may be held by the second holding mechanism. Accordingly, an outer surface of the large-diameter housing portion 82 is exposed, and a portion at which the sealing cap is gripped by hand is enlarged, and the sealing cap can be readily removed.

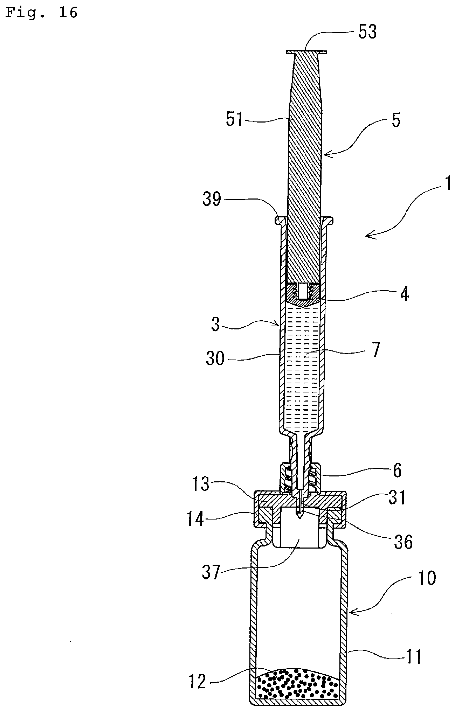

Next, operation of the syringe barrel with puncture function and the prefilled syringe with puncture function according to the present invention will be described using Figs. 16 to 19.

When the medical liquid filled in the prefilled syringe with puncture function according to the present invention is directly administered, the cap 8 is removed from the prefilled syringe 1 with puncture function, and the prefilled syringe 1 is brought into a state illustrated in Figs. 3 and 4. The syringe barrel 3 according to the present invention has a peculiar form having the small-diameter distal end portion 32 and the large-diameter portion 33, at the distal end portion. Thus, the differences between the syringe barrel 3 and a conventional syringe can be readily recognized, and the syringe barrel 3 has a low probability of incorrect administration.

The large-diameter portion of the syringe barrel 3 is connected to the connection portion of another medical device having a female type luer taper. When the connection portion of the medical device can be connected to the lock adapter 6, the lock adapter 6 is used while the large-diameter portion is covered by the lock adapter, as illustrated in Figs. 3 and 4. In contrast, when the connection portion of the medical device cannot be connected to the lock adapter 6, the lock adapter 6 is proximally moved to expose the small-diameter distal end portion and the large-diameter portion of the distal end portion of the syringe barrel 3, as illustrated in Figs. 5 and 6. Thus, the large-diameter portion can be connected to the connection portion of the medical device without influence of the lock adapter 6.

Connection between the lock adapter 6 and the medical device (injection needle) 20 capable of being connected to the lock adapter 6 brings about a state illustrated in Fig. 15. In the state illustrated in Fig. 15, the hub 21 of the injection needle 20 is liquid-tightly connected to the large-diameter portion 33, and the rear end external rib 23 of the hub 21 is threadedly engaged with the thread engagement portion 67 of the lock adapter 6. Furthermore, the small-diameter distal end portion 32 is housed in the hub 21. The prefilled syringe 1 to which the injection needle 20 is connected allows administration performed by directly puncturing a human, similar to a conventional syringe. The inside of the hub 21 may have a short length Z of approximately 8.6 mm in an insertion direction of the large-diameter portion 33. Even in this configuration, since the outer diameter of the distal end of the large-diameter portion 33 is 4.04 to 4.20 mm, and the length of the small-diameter distal end portion 32 is 5 to 6 mm, the small-diameter distal end portion 32 can be housed in the hub 21, while the large-diameter portion 33 is liquid-tightly connected to the hub 21. Furthermore, the thread engagement between the thread engagement portion 67 of the lock adapter 6 and the rear end external rib 23 of the hub 21 fixedly connects the hub 21 and the large-diameter portion 33. Thus, even if the length of the large-diameter portion 33 inserted into the hub 21 is small, and a fitting force between the large-diameter portion 33 and the hub 21 is less than a fitting force between a needle cover (not illustrated) covering the needle 22 of the injection needle 20 and the hub 21, disconnection between the large-diameter portion 33 and the hub 21 does not occur when the needle cover is removed from the hub 21.

Furthermore, when the prefilled syringe 1 according to the present invention is connected to the medication container 10 such as the vial, similarly to the above description, the cap 8 is removed from the prefilled syringe 1 with puncture function, and the prefilled syringe 1 is brought into the state illustrated in Figs. 3 and 4. The medication container 10 includes a container body 11, a medication 12 stored in the container body 11, a rubber plug 13 configured to seal an opening portion of the container body 11, and a fixing portion 14 configured to fix the rubber plug 13 in the opening portion of the medication container body 11. When the rubber plug 13 of the medication container 10 allows the lateral side holes 36 to reach the inside of the medication container 10 only by puncture of the small-diameter portion 32 of the prefilled syringe 1with puncture function, the prefilled syringe is used without proximally moving the lock adapter 6. Then, as illustrated in Fig. 16, the distal end portion 31 of the syringe barrel 3 pierces the rubber plug 13 from the puncture end portion 37 of the small-diameter distal end portion 32, and the distal end portion 31 and the lateral side holes 36 reach the inside of the medication container 10.

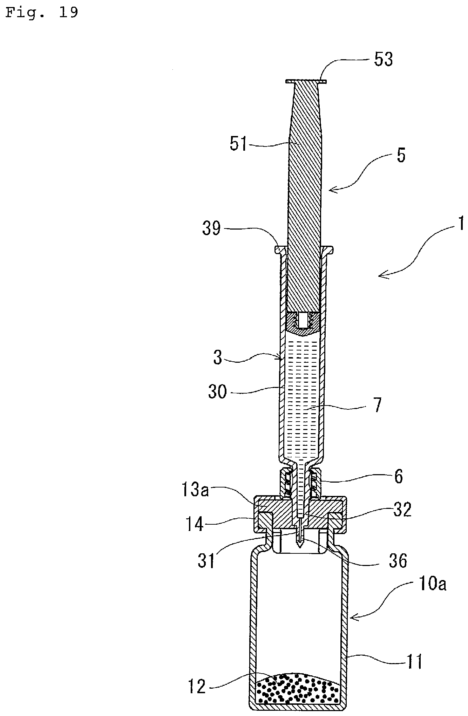

As in a medication container 10a illustrated in Fig. 19, when the medication container has a thick rubber plug 13a, the lateral side holes 36 may not reach the inside of the medication container 10a only by puncture of the small-diameter portion 32 of the prefilled syringe 10 with puncture function. In such a configuration, the distal end portion 31 of the prefilled syringe 1 with puncture function is pushed into the rubber plug, and the lock adapter 6 is proximally moved, as illustrated in Fig. 19. Then, as illustrated in Fig. 19, not only the small-diameter portion 32 of the distal end portion 31 of the syringe barrel pierces the medication container 10a, but also the large-diameter portion 33 pierces the rubber plug 13a of the medication container 10a, and the lateral side holes 36 in the small-diameter portion 32 reach the inside of the medication container. Thus, the medical liquid (solvent) 7 in the syringe 1 can be injected into the medication container 10a. The distal end portion 31 is pushed into the rubber plug until the projection 64 of the lock adapter moves over the rib 73 of the sliding portion 34 to be positioned at the recessed portion 74, and then the pushing is finished. Thus, the user can confirm the finish of the pushing of the distal end portion 31 into the rubber plug, based on touch or sound when the projection 64 moves over the rib 73.

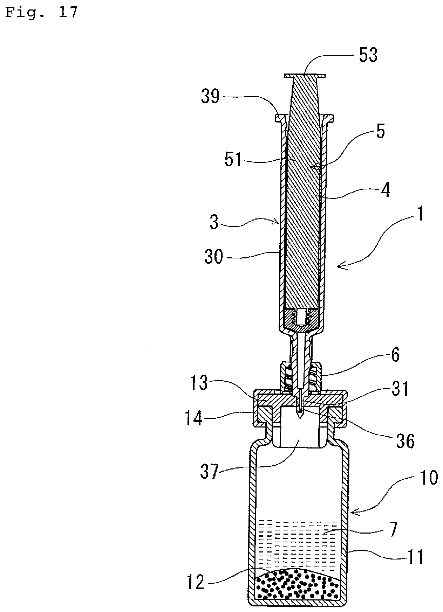

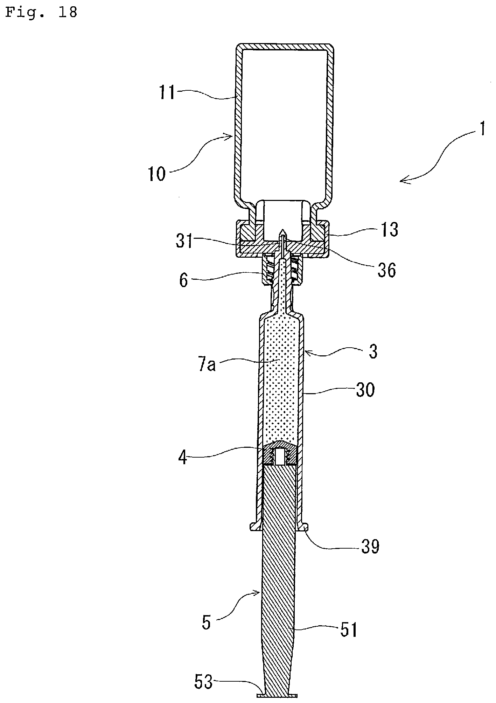

In a state illustrated in Fig. 16, the plunger 5 of the syringe 1 is pushed, and the medical liquid (solvent) 7 in the syringe 1 flows into the medication container 10. The above state is illustrated in Fig. 17. After the medical liquid 7 and the medication 12 are mixed, the medication container 10 is positioned above, the plunger 5 of the syringe 1 is drawn, and medication dissolved solution 7a in the medication container is collected in the syringe, as illustrated in Fig. 18. Similarly to Fig. 15, the prefilled syringe 1 is connected to an injection needle, a port of the three-way stopcock as general administration means, and the direct administration can be allowed.

Note that the syringe barrel with puncture function and the prefilled syringe with puncture function according to an embodiment of the present invention preferably include the above-mentioned lock adapter, but in other embodiments, may not include the lock adapter 6. In embodiments that do not include the lock adapter 6, the syringe barrel 3 with puncture function may not include the sliding portion 34.

Furthermore, although the lock adapter 6 preferably slides, the lock adapter 6 may be fixed to a position on the distal end side so that the small-diameter portion 32 of the distal end portion 31 of the syringe barrel is exposed, and the large-diameter portion 33 is substantially covered, as illustrated in Figs. 3 and 4. In this configuration, the syringe barrel 3 with puncture function may not include the sliding portion 34.

### Industrial Applicability

The syringe barrel with puncture function according to the present invention is achieved by the followings.
(1) A syringe barrel with puncture function including a cylindrical main body portion configured to allow liquid-tight sliding of a gasket, and a hollow distal end portion configured to extend distally from the cylindrical main body portion. The distal end portion of the syringe barrel includes a hollow small-diameter distal end portion having a puncture end portion configured to be inserted into a puncturable portion of another medical device, and a large-diameter portion configured to have a diameter larger than that of the small-diameter distal end portion. The outer diameter of the large-diameter portion is increased from a distal end to a proximal end thereof by a 6/100 taper. The distal end of the large-diameter portion has an outer diameter of 4.04 to 4.20 mm, and the small-diameter distal end portion has a length of 5 to 6 mm. The small-diameter distal end portion can be used to puncture a puncturable portion of the medical device, and the large-diameter portion can be used to connect to a connection portion of other medical device having a female luer taper. The small-diameter distal end portion is housed in the connection portion, upon connection of the large-diameter portion to the connection portion, and the large-diameter portion has a length of 5 to 9 mm.

Thus, the cylindrical main body portion can puncture the puncturable portion of the medical device, using the small-diameter distal end portion. The outer diameter at the distal end portion of the large-diameter portion is large, and the large-diameter portion can be liquid-tightly connected to the connection portion of the medical device having a female luer taper, but the length of the large-diameter portion inserted into the connection portion is small, and some space remains in the connection portion distally from the large-diameter portion. The small-diameter portion has a small total length, and can be housed in the space. Thus, in spite of the small-diameter portion, connection can be made to the connection portion of the medical device having a female luer taper, using the large-diameter portion.

## Claims

1. A syringe barrel (3) with puncture function comprising:
a cylindrical main body portion (30) configured to allow liquid-tight sliding of a gasket (4); and
a hollow distal end portion (31) configured to extend distally from the cylindrical main body portion (30),
the distal end portion (31) of the syringe barrel (3) including:
a hollow small-diameter distal end portion (32) having a puncture end portion (37) configured to be inserted into a puncturable portion of a medical device; and
a large-diameter portion (33) having a diameter larger than a diameter of the small-diameter distal end portion (32), an outer surface of the large-diameter portion (33) being tapered such that the outer diameter of the large-diameter portion (33) increases from a distal end to a proximal end thereof by a 6/100 taper, **characterized by**
the distal end of the large-diameter portion (33) having an outer diameter (X) of 4.04 to 4.20 mm, the small-diameter distal end portion (32) having a length (Y) of 5 to 6 mm, the small-diameter distal end portion (32) capable of being used to puncture a puncturable portion of the other medical device, and the large-diameter portion (33) is configured to connect to a connection portion of a medical device having a female luer taper, and the small-diameter distal end portion (32) is housed in the connection portion upon connection of the large-diameter portion (33) to the connection portion, and the large-diameter portion (33) has a length of 5 to 9 mm.

2. The syringe barrel (3) with puncture function according to claim 1,
wherein the puncture end portion (37) of the small-diameter distal end portion (32) is formed by a closed pointed end, and the small-diameter distal end portion (32) includes a lateral side hole (36) configured to communicate between an interior and an exterior of the small-diameter distal end portion (32).

3. The syringe barrel (3) with puncture function according to claim 1 or 2, further comprising a lock adapter (6) configured to hold the connection portion of the medical device, the distal end portion (31) of the syringe barrel (3) includes a lock adapter sliding portion (34) provided between the cylindrical main body portion (30) and the large-diameter portion (33), the lock adapter (6) being slidably mounted to the sliding portion (34).

4. The syringe barrel (3) with puncture function according to claim 3,
wherein the lock adapter (6) includes, on an inner surface, an adapter-side engagement portion (67) configured to be engaged with a connection-side projection portion provided at the connection portion of the medical device.

5. The syringe barrel (3) with puncture function according to claim 3 or 4,
wherein the lock adapter (6) is axially slidable from a state in which the large-diameter portion (33) is substantially covered by the lock adapter (6), to a state in which the large-diameter portion (33) is substantially exposed.

6. The syringe barrel (3) with puncture function according to any of claims 3 to 5,
wherein the lock adapter (6) is mounted to the sliding portion (34) in a slidable and non-rotatable manner.

7. The syringe barrel (3) with puncture function according to any of claims 3 to 6,
wherein the lock adapter (6) and the sliding portion (34) include a first holding mechanism configured to hold the lock adapter (6) in a state in which the lock adapter (6) substantially covers the large-diameter portion (33), and a second holding mechanism configured to hold the lock adapter (6) in a state in which the large-diameter portion (33) is substantially exposed.

8. The syringe barrel (3) with puncture function according to any of claims 1 to 7, wherein the distal end of the large-diameter portion (33) has an outer diameter of 4.04 to 4.16 mm.

9. A syringe (2) with puncture function comprising:
the syringe barrel (3) with puncture function according to any of claims 1 to 8;
a gasket (4) configured to be slidably housed in the barrel (3); and
a pusher (5) configured to be mounted to or mountable to a rear end of the gasket (4).

10. A prefilled syringe (1) with puncture function comprising:
the syringe (2) with puncture function according to claim 9;
a medical liquid (7) contained in the syringe barrel (3); and
a sealing cap (8) configured to seal the distal end portion (31) of the barrel (3).

## Patentansprüche

1. Spritzenzylinder (3) mit Durchstechfunktion, umfassend:
einen zylindrischen Hauptkörperabschnitt (30), der so konfiguriert ist, dass er ein flüssigkeitsdichtes Gleiten einer Dichtung (4) ermöglicht; und
einen hohlen distalen Endabschnitt (31), der so konfiguriert ist, dass er sich distal von dem zylindrischen Hauptkörperabschnitt (30) erstreckt,
wobei der distale Endabschnitt (31) des Spritzenzylinders (3) umfasst:
einen hohlen distalen Endabschnitt (32) mit kleinem Durchmesser, der einen Durchstechendabschnitt (37) aufweist, der so konfiguriert ist, dass er in einen durchstechbaren Abschnitt einer medizinischen Vorrichtung eingeführt werden kann; und
einen Abschnitt (33) mit großem Durchmesser, der einen Durchmesser aufweist, der größer als ein Durchmesser des distalen Endabschnitts (32) mit kleinem Durchmesser ist, wobei eine Außenfläche des Abschnitts (33) mit großem Durchmesser derart verjüngt ist, dass der Außendurchmesser des Abschnitts (33) mit großem Durchmesser von einem distalen Ende zu einem proximalen Ende desselben durch eine 6/100-Verjüngung zunimmt, **gekennzeichnet dadurch**
**dass** das distale Ende des Abschnitts (33) mit großem Durchmesser einen Außendurchmesser (X) von 4,04 bis 4,20 mm aufweist, der distale Endabschnitt (32) mit kleinem Durchmesser eine Länge (Y) von 5 bis 6 mm aufweist, wobei der distale Endabschnitt (32) mit kleinem Durchmesser zum Durchstechen eines durchstechbaren Abschnitts der anderen medizinischen Vorrichtung verwendet werden kann und der Abschnitt (33) mit großem Durchmesser zum Verbinden mit einem Verbindungsabschnitt einer medizinischen Vorrichtung konfiguriert ist, die einen weiblichen Luer-Konus aufweist, und der distale Endabschnitt (32) mit kleinem Durchmesser beim Verbinden des Abschnitts (33) mit großem Durchmesser mit dem Verbindungsabschnitt in dem Verbindungsabschnitt untergebracht ist, und der Abschnitt (33) mit großem Durchmesser eine Länge von 5 bis 9 mm aufweist.

2. Spritzenzylinder (3) mit Durchstechfunktion nach Anspruch 1,
wobei der Durchstechendabschnitt (37) des distalen Endabschnitts (32) mit kleinem Durchmesser durch ein geschlossenes spitzes Ende gebildet ist und der distale Endabschnitt (32) mit kleinem Durchmesser ein laterales Seitenloch (36) aufweist, das konfiguriert ist, um zwischen einem Inneren und einem Äußeren des distalen Endabschnitts (32) mit kleinem Durchmesser zu kommunizieren.

3. Spritzenzylinder (3) mit Durchstechfunktion nach Anspruch 1 oder 2, ferner umfassend einen Verriegelungsadapter (6), der zum Halten des Verbindungsabschnitts der medizinischen Vorrichtung konfiguriert ist, wobei der distale Endabschnitt (31) des Spritzenzylinders (3) einen Verriegelungsadapter-Gleitabschnitt (34) aufweist, der zwischen dem zylindrischen Hauptkörperabschnitt (30) und dem Abschnitt (33) mit großem Durchmesser vorgesehen ist, wobei der Verriegelungsadapter (6) verschiebbar an dem Gleitabschnitt (34) angebracht ist.

4. Spritzenzylinder (3) mit Durchstechfunktion nach Anspruch 3,
wobei der Verriegelungsadapter (6) auf einer Innenfläche einen adapterseitigen Eingriffsabschnitt (67) aufweist, der so konfiguriert ist, dass er mit einem verbindungsseitigen Vorsprungsabschnitt, der an dem Verbindungsabschnitt der medizinischen Vorrichtung vorgesehen ist, in Eingriff gebracht wird.

5. Spritzenzylinder (3) mit Durchstechfunktion nach Anspruch 3 oder 4,
wobei der Verriegelungsadapter (6) von einem Zustand, in dem der Abschnitt (33) mit großem Durchmesser im Wesentlichen durch den Verriegelungsadapter (6) abgedeckt ist, in einen Zustand axial verschiebbar ist, in dem der Abschnitt (33) mit großem Durchmesser im Wesentlichen freigelegt ist.

6. Spritzenzylinder (3) mit Durchstechfunktion nach einem der Ansprüche 3 bis 5,
wobei der Verriegelungsadapter (6) an dem Gleitabschnitt (34) in einer verschiebbaren und nicht drehbaren Weise angebracht ist.

7. Spritzenzylinder (3) mit Durchstechfunktion nach einem der Ansprüche 3 bis 6,
wobei der Verriegelungsadapter (6) und der Gleitabschnitt (34) einen ersten Haltemechanismus umfassen, der so konfiguriert ist, dass er den Verriegelungsadapter (6) in einem Zustand hält, in dem der Verriegelungsadapter (6) im Wesentlichen den Abschnitt (33) mit großem Durchmesser abdeckt, und einen zweiten Haltemechanismus, der so konfiguriert ist, dass er den Verriegelungsadapter (6) in einem Zustand hält, in dem der Abschnitt (33) mit großem Durchmesser im Wesentlichen freigelegt ist.

8. Spritzenzylinder (3) mit Durchstechfunktion nach einem der Ansprüche 1 bis 7, wobei das distale Ende des Abschnitts (33) mit großem Durchmesser einen Außendurchmesser von 4,04 bis 4,16 mm aufweist.

9. Spritze (2) mit Durchstechfunktion, umfassend:
den Spritzenzylinder (3) mit Durchstechfunktion nach einem der Ansprüche 1 bis 8;
eine Dichtung (4), die so konfiguriert ist, dass sie in dem Zylinder (3) verschiebbar untergebracht ist; und
einen Schieber (5), der so konfiguriert ist, dass er an einem hinteren Ende der Dichtung (4) angebracht ist oder anbringbar ist.

10. Vorgefüllte Spritze (1) mit Durchstechfunktion, umfassend:
die Spritze (2) mit Durchstechfunktion nach Anspruch 9;
eine medizinische Flüssigkeit (7), die in dem Spritzenzylinder (3) enthalten ist; und
eine Verschlusskappe (8), die so konfiguriert ist, dass sie den distalen Endabschnitt (31) des Zylinders (3) verschließt.

## Revendications

1. Corps (3) de seringue à fonction de perforation, comportant :
un tube cylindrique principal (30) conçu pour permettre un coulissement d'un joint (4) sans fuites de fluide ; et
une extrémité distale creuse (31) conçue pour s'étendre distalement depuis le tube cylindrique principal (30),
l'extrémité distale (31) du corps (3) de seringue comprenant :
une partie creuse (32) à petit diamètre d'extrémité distale, ayant un bout perforant (37) conçu pour être inséré dans une partie perforable d'un dispositif médical ; et
une partie (33) à grand diamètre ayant un diamètre plus grand qu'un diamètre de la partie (32) à petit diamètre d'extrémité distale, une surface extérieure de la partie (33) à grand diamètre étant conique de telle manière que le diamètre extérieur de la partie (33) à grand diamètre augmente depuis une extrémité distale jusqu'à une partie proximale suivant une conicité de 6/100, **caractérisé en ce que**
l'extrémité distale de la partie (33) a grand diamètre a un diamètre extérieur (X) de 4,04 à 4,20 mm, la partie (32) à petit diamètre d'extrémité distale ayant une longueur (Y) de 5 à 6 mm, la partie (32) à petit diamètre d'extrémité distale étant apte à perforer une partie perforable de l'autre dispositif médical, et la partie (33) à grand diamètre est conçue pour se raccorder à un moyen de raccordement d'un dispositif médical a conicité Luer femelle, et la partie (32) à petit diamètre d'extrémité distale est logée dans le moyen de raccordement au moment du raccordement de la partie (33) à grand diamètre au moyen de raccordement, et la partie (33) à grand diamètre a une longueur de 5 à 9 mm.

2. Corps (3) de seringue à fonction de perforation selon la revendication 1, dans lequel le bout perforant (37) de la partie (32) à petit diamètre d'extrémité distale est formé par une extrémité pointue fermée, et la partie (32) à petit diamètre d'extrémité distale comprend un trou latéral (36) conçu pour faire communiquer un intérieur et un extérieur de la partie (32) à petit diamètre d'extrémité distale.

3. Corps (3) de seringue à fonction de perforation selon la revendication 1 ou 2, comportant en outre un adaptateur verrouillant (6) conçu pour retenir le moyen de raccordement du dispositif médical, l'extrémité distale (31) du corps (3) de seringue comportant une partie coulissante (34) de moyen de raccordement présente entre le tube cylindrique principal (30) et la partie (33) à grand diamètre, l'adaptateur verrouillant (6) étant monté pour pouvoir coulisser sur la partie coulissante (34).

4. Corps (3) de seringue à fonction de perforation selon la revendication 3,
dans lequel l'adaptateur verrouillant (6) comprend, sur une surface intérieure, un moyen d'enclenchement (67) côté adaptateur conçu pour être enclenché avec une partie formant saillie côté raccordement présente sur le moyen de raccordement du dispositif médical.

5. Corps (3) de seringue à fonction de perforation selon la revendication 3 ou 4,
dans lequel l'adaptateur verrouillant (6) peut coulisser axialement d'une position dans laquelle la partie (33) à grand diamètre est sensiblement couverte par l'adaptateur verrouillant (6) à une position dans laquelle la partie (33) à grand diamètre est sensiblement découverte.

6. Corps (3) de seringue à fonction de perforation selon l'une quelconque des revendications 3 à 5,
dans lequel l'adaptateur verrouillant (6) est monté sur la partie coulissante (34) d'une manière permettant le coulissement et empêchant la rotation.

7. Corps (3) de seringue à fonction de perforation selon l'une quelconque des revendications 3 à 6,
dans lequel l'adaptateur verrouillant (6) et la partie coulissante (34) comprennent un premier mécanisme de retenue conçu pour retenir l'adaptateur verrouillant (6) dans une position dans laquelle l'adaptateur verrouillant (6) couvre sensiblement la partie (33) à grand diamètre, et un second mécanisme de retenue conçu pour retenir l'adaptateur verrouillant (6) dans une position dans laquelle la partie (33) à grand diamètre est sensiblement découverte.

8. Corps (3) de seringue à fonction de perforation selon l'une quelconque des revendications 1 à 7, dans lequel l'extrémité distale de la partie (33) à grand diamètre a un diamètre extérieur de 4,04 à 4,16 mm.

9. Seringue (2) à fonction de perforation, comportant :
le corps (3) de seringue à fonction de perforation selon l'une quelconque des revendications 1 à 8,
un joint (4) conçu pour être logé dans le corps (3) de manière à pouvoir coulisser ; et
un piston (5) conçu pour être monté sur ou montable à une extrémité arrière du joint (4).

10. Seringue préremplie (1) à fonction de perforation, comportant :
la seringue (2) à fonction de perforation selon la revendication 9 ;
un liquide médical (7) contenu dans le corps (3) de seringue ; et
un capuchon d'obturation (8) conçu pour obturer l'extrémité distale (31) du corps (3).
